Europäisches Patentamt

European Patent Office

Office européen des brevets

(11) Publication number: **0 289 478**
**A2**

# EUROPEAN PATENT APPLICATION

(21) Application number: 88870069.7

(22) Date of filing: 26.04.88

(51) Int. Cl.⁴: **C 12 N 15/00**
A 01 H 1/00, C 12 N 5/00,
C 12 N 1/20
// C12N9/10

(30) Priority: **27.04.87 US 42916**

(43) Date of publication of application:
**02.11.88 Bulletin 88/44**

(84) Designated Contracting States:
**AT BE CH DE ES FR GB GR IT LI LU NL SE**

(71) Applicant: **Monsanto Company**
**Patent Department 800 North Lindbergh Boulevard**
**St. Louis Missouri 63167 (US)**

(72) Inventor: **Rogers, Stephen Gary**
**14788 Timberbluff**
**Chesterfield Missouri 63017 (US)**

**Klee, Harry John**
**802 Rotherham Drive**
**Ballwin Missouri 63011 (US)**

**Hayford, Maria Burmaz**
**1530 Timber Point Court**
**Chesterfield Missouri 63017 (US)**

(74) Representative: **Lunt, John Cooper et al**
**Monsanto Europe S.A. Patent Department Avenue de**
**Tervuren 270-272 Letter Box No 1**
**B-1150 Brussels (BE)**

(54) Gentamicin marker genes for plant transformation.

(57) An improved method for selecting transformed plant cells is disclosed. The invention involves the use of a selectable plant marker gene encoding a gentamicin-3-N-acetyltransferase enzyme. The use of the gentamicin marker genes affords advantages over known markers in some plants. The invention also provides transformed plant cells that contain the gentamicin marker genes as well as differentiated plants containing transformed plant cells.

EP 0 289 478 A2

**Description**

## GENTAMICIN MARKER GENES FOR PLANT TRANSFORMATION

The present invention relates to genetic engineering and plant transformation. More particularly, the present invention relates to an improvement which comprises using a marker gene for selecting transformed plant cells, said gene encoding a gentamicin-3-N-acetyltransferase (AAC(3)) enzyme and adapted to express the enzyme at a sufficient level to render transformed plant tissue tolerant of normally lethal levels of gentamicin. The use of the gentamicin marker genes of the present invention exhibits advantages over known markers in some plants.

A variety of methods are known for introducing exogenous DNA into plant cells. Such methods include protoplast fusion, microinjection, liposomes to encapsulate DNA and the contacting of plant cells with DNA complexed with calcium phosphate and polycationic substances. At present a widely used system employs the bacterium *Agrobacterium tumefaciens* which is known to transform plant cells via the T-DNA of the Ti (tumor inducing) plasmid (Bevan et al., 1982; Depicker et al., 1983). A small fragment of the Ti plasmid, called T-DNA (transferred DNA), is stably integrated into plant nuclear DNA and is actively transcribed. Specific genes of the T-DNA cause high levels of phytohormone production and crown gall formation. Elevated phytohormone levels have largely prevented regeneration of whole plants from transformed cells. As a result, disarmed Ti plasmids which lack the phytohormone genes have been engineered to induce plant transformation (Horsch et al., 1985).

In order to identify the plant cells which have been transformed in the absence of phytohormone selection, dominant selectable marker genes have been inserted into the T-DNA. One such widely used marker gene is the neomycin phosphotransferase, type II gene (NPTII). This bacterial gene confers resistance to normally lethal levels of several aminoglycosidic compounds in a variety of eucaryotic organisms when fused to appropriate transcriptional elements. For expression in plants, the NPTII gene is usually fused to the promoter and 3' non-translated region of the nopaline synthase gene (Fraley et al., 1985). This chimeric gene (NOS/NPTII/NOS) is widely used to select transformed plant cells on kanamycin and G418 antibiotics.

Unfortunately, the NOS/NPTII/NOS gene does not confer a phenotype which is easily selectable in all plant species. For example, cultivated alfalfa transformants cannot be easily selected using kanamycin resistance as a marker. Therefore, in order to make transformation of this commercially important legume and other plants more practical, alternative selectable markers are needed. Such markers are also useful in co-transformation systems in which transformed plant tissue is transformed again with a plant transformation vector carrying a second selectable marker and, in most cases, additional genes of interest.

BRIEF DESCRIPTION OF THE DRAWINGS

Figure 1 shows the DNA and amino acid sequence for the AAC(3)-IV enzyme of Example 1.
Figure 2 shows the steps employed in the preparation of plasmid pMON825.
Figure 3 shows a plasmid map for pMON316.
Figure 4 shows a plasmid map for pMON200.
Figure 5 shows the DNA sequence for the CaMV35S, synthetic multi-linker and NOS 3' polyadenylation signal used in the constructs of the present invention.
Figure 6 shows a plasmid map for plant transformation vector pMON505.
Figure 7 shows a plasmid map for plant transformation vector pMON530.
Figure 8 shows the DNA and amino acid sequence of the AAC(3)-III enzyme of Example 2.
Figure 9 shows the steps employed in the preparation of plasmid pMON851.
Figure 10 shows the steps employed in the preparation of plasmid pMON857.

STATEMENT OF THE INVENTION

The present invention contemplates the use of plant genes which encode gentamicin-3-N-acetyl transferase as selectable markers in plant transformation. Gentamicin-3-N-acetyltransferase enzymes inactivate certain aminoglycosidic compounds by acetylation of the amino group in position 3 of the 2-deoxystreptamine moiety (Brau et al., 1984). This group of enzymes, commonly called AAC(3) enzymes since they acetylate the 3-amino group of glycosidic compounds, are often subdivided on the basis of *in vivo* resistance spectrum, *in vitro* substrate profiles and isoelectric point determination. To date, four classes of AAC(3) enzymes have been identified.

AAC(3)-I has the most limited range of substrates of any of the four classes of AAC(3) enzymes. AAC(3)-I was first isolated from a strain of *Pseudomonas aeruginosa* (Brzezinska et al., 1972) and has subsequently been isolated from clinical isolates of *E. coli* (Umezawa et al., 1973; Witchitz, 1972). It has been shown that the gentamicin C antibiotics and sisomicin are excellent substrates for the enzyme, while tobramycin, kanamycin A, B and C and gentamicin A are either poor substrates or are not acetylated (Brzezinska et al., 1972). AAC(3)-I enzymes have a molecular weight of about 63,000±6300 and are composed of four identical subunits of molecular weight 17,000 ±1700. The enzymes have a broad pH activity ranging from 5.5 to 8.5, an isoelectric point (pI) of about 7.4 and are stable to lyophilization and storage at -20°C (Williams et al., 1976).

AAC(3)-II was first isolated from a *Klebsiella sp* (LeGoffic et al., 1974). On the basis of its *in vitro* substrate profile it appears very similar to AAC(3)-I enzymes, but has a pI of about 6.4.

AAC(3)-III enzymes have a much wider substrate range than AAC(3)-I enzymes. Substrates include the gentamicins, sisomicin, netilmicin, kanamycins, tobramycin, neomycin and paromomycin. AAC(3)-III enzymes have been found in numerous clinical isolates (Rosenthal et al., 1976).

AAC(3)-IV enzymes have the broadest range of substrates of all AAC(3) enzymes. AAC(3)-IV enzymes acetylate all of the substrates of AAC(3)-III enzymes in addition to the aminoglycoside apramycin (Davies et al., 1978).

Bacteria containing gentamicin-3-N-acetyltransferase enzymes can be easily isolated using conventional microbiological techniques. A bacterial innoculant is plated on appropriate nutrient media containing normally inhibitory levels of gentamicin. Concentrations between about 5 and 25 μg/ml gentamicin are usually inhibitory for most bacteria. Resistant bacteria containing genes which express gentamicin-3-N-acetyltransferase are capable of growing on such a medium. A colony of resistant bacteria is propagated, and purified to homogenity by serially cloning bacteria from the identified resistant colony. A clone bank is made from randomly sheared DNA of the resistant bacteria in a plasmid capable of replication in *E. coli* bacteria. Preferably the plasmid used to construct the clone bank should also contain a broad host range plasmid origin of replication so that the plasmid can be mobilized to other gram-negative bacteria. Exemplary of such broad host range origins of replication are those of the P1 incompatibility group (e.g. RK2/RP4) or the Q incompatibility group (e.g. RSF1010). The clone bank is transformed into *E. coli* and a portion of this bank is plated out on appropriate nutrient media containing a level of gentamicin toxic to normal *E. coli* cells. Concentrations between about 10 μg/ml and 20 μg/ml gentamicin should be effective for this selection process. Any bacteria which grow on the selective medium should contain a clone of the gentamicin-3-N-acetyltransferase gene. This is verified by reisolating the plasmid and introducing it into a new *E. coli* to demonstrate co-transfer of the plasmid and the attendant resistance to gentamicin.

If no *E. coli* are found that are resistant to gentamicin, the gene may not confer resistance in *E. coli*. In this case, the entire clone bank is mobilized into another gram-negative bacterium such as a *Pseudomonas sp* and selected on gentamicin-containing medium as described above. Plasmids can be mobilized from *E. coli* to a *Pseudomonas sp.* by triparental mating using the helper plasmid pRK2013 (Ditta et al., 1980). The clone bank is preferably mobilized into the organism from which the gene was originally isolated. In this manner, a DNA clone conferring gentamicin resistance can be identified.

Once a plasmid conferring gentamicin resistance has been identified, the gentamicin-3-N-acetyltransferase gene can be identified by subcloning restriction endonuclease fragments which continue to confer gentamicin resistance. At this point, the DNA sequence of this region is determined and the area encoding the gene identified. The correct coding sequence can be unambiguously defined by mutational analysis of the DNA within and surrounding the proposed gene. Mutational analysis comprises deletion of a portion of the gene and demonstration of the attendant loss of gene activity.

When the coding sequence has been defined, the region encoding the gentamicin-3-N-acetyltransferase enzyme is cloned into a vector designed for expression of genes in plants. Such a transformation vector would consist of a transcriptional promoter element active in plants and a polyadenylation signal sequence with a suitable restriction endonuclease recognition site between them. The gentamicin-3-N-acetyltransferase coding sequence is placed into the restriction site in an orientation which permits the promoter element to synthesize a mRNA encoding the gentamicin-3-N-acetyltransferase enzyme.

In one aspect, the present invention provides a selectable plant marker gene comprising in sequence:

(a) a promoter which functions in plant cells to cause the production of RNA;

(b) a DNA coding sequence that causes the production of RNA encoding a bacterial gentamicin-3-N-acetyltransferase enzyme; and

(c) a 3' non-translated region which functions in plant cells to cause the addition of polyadenylate nucleotides to the 3' end of the RNA.

Transcription of DNA into mRNA is regulated by a region of DNA usually referred to as the "promoter." The promoter region contains a sequence of bases which signals RNA polymerase to associate with the DNA, and initiated the transcription of messenger RNA using one of the DNA strands as a template to make a corresponding strand of RNA. A number of promoters which are active in plant cells have been described in the literature, including the nopaline synthase (NOS), octopine synthase (OCS) and mannopine synthase (MAS) promoters (which are carried on tumor inducing plasmids of *Agrobacterium tumefaciens*), the cauliflower mosaic virus (CaMV) 19S and 35S promoters, and the light-inducible promoter from the small subunit of ribulose bis-phosphate carboxylase (ssRUBISCO), a very abundant plant polypeptide. All of these promoters have been used to create various types of chimeric genes which have been expressed in plants; see e.g., PCT publication WO 84/02913 (Rogers et al., Monsanto).

Promoters which are known or found to cause the production of bacterial RNA in plant cells can be used in the present invention. Such promoters may be obtained from plants or plant viruses and include, but are not necessarily limited to, the CaMV19S and CaMV35S promoters and promoters isolated from plant genes such as ssRUBISCO genes. The particular promoter selected should be capable of causing sufficient expression of gentamicin-3-N-acetyltransferase to render the plant substantially tolerant of normally lethal levels of gentamicin. Those skilled in the art will recognize that the amount of gentamicin-3-N-acetyltransferase needed to induce tolerance may vary with the type of plant. Accordingly, while the CaMV35S promoter is preferred it should be understood that this promoter may not be the optimal promoter for all embodiments of the present invention.

3

The 3′ non-translated region contains a polyadenylation signal which functions in plants to cause the addition of polyadenylation nucleotides to the 3′ end of the RNA transcript. Examples of suitable 3′ regions are the 3′ transcribed, non-translated regions containing the polyadenylation signal of the nopaline synthase (NOS) gene of the *Agrobacterium* tumor-inducing (Ti) plasmid or the conglycinin (7S) storage protein gene. An example of a preferred 3′ region is that from the NOS gene, described in the examples hereinafter.

The RNA produced by the chimeric gene also contains a 5′ non-translated leader sequence. This sequence may be derived from the promoter selected to express the gene and may be specifically modified so as to increase translation of the mRNA. The 5′ non-translated regions may also be obtained from plant viruses, other suitable eukaryotic genes or a synthetic gene sequence.

In another aspect, the present invention provides a method for selecting a transformed plant cell which comprises:

(a) inserting DNA comprising a plant gene encoding a gentamicin-3-N-acetyltransferase enzyme into the genome of the plant cell; and

(b) selecting a plant cell containing said gene by culturing said cell in the presence of normally lethal levels of gentamicin.

The gentamicin marker genes of the present invention are inserted into the genome of a plant by any suitable method. Suitable plant transformation vectors include those derived from a Ti plasmid of *Agrobacterium tumefaciens* as well as those described in e.g. Herrera-Estrella 1983, Bevan 1983, Klee 1985 and EPO publication 120,516 (Schilperoort et al.). In addition to plant transformation vectors derived from the Ti or root-inducing (Ri) plasmids of *Agrobacterium*, alternative methods can be used to insert the chimeric genes of this invention into plant cells. Such methods may involve, for example, liposomes, electroporation, chemicals which increase free DNA uptake, and the use of viruses or pollen as vectors.

The transformed plant cell is then directly selected from untransformed cells by growth on medium containing normally lethal levels of gentamicin. The preferred range of gentamicin is between about 25 μg/ml and 300 μg/ml and more preferably about 50 μg/ml to about 150 μg/ml. Those skilled in the art will recognize that the optimal concentration which con trols the escape of untransformed cells while permitting substantial growth of transformed plant cells will, in many cases, vary with the plant species.

There has also been provided, in accordance with another aspect of the present invention, bacterial cells, co-integrating *Agrobacterium*-based vectors, binary *Agrobacterium*-based vectors and transformed plant cells that contain the above-described gentamicin marker gene. In yet another aspect, the present invention provides a differentiated plant that comprises transformed plant cells, as described above, which exhibits tolerance to normally lethal levels of gentamicin.

The following examples are provided to better elucidate the practice of the present invention. It should be understood that these examples are provided for illustrative purposes only, and are not intended to limit the scope of the invention in any way.

Example 1

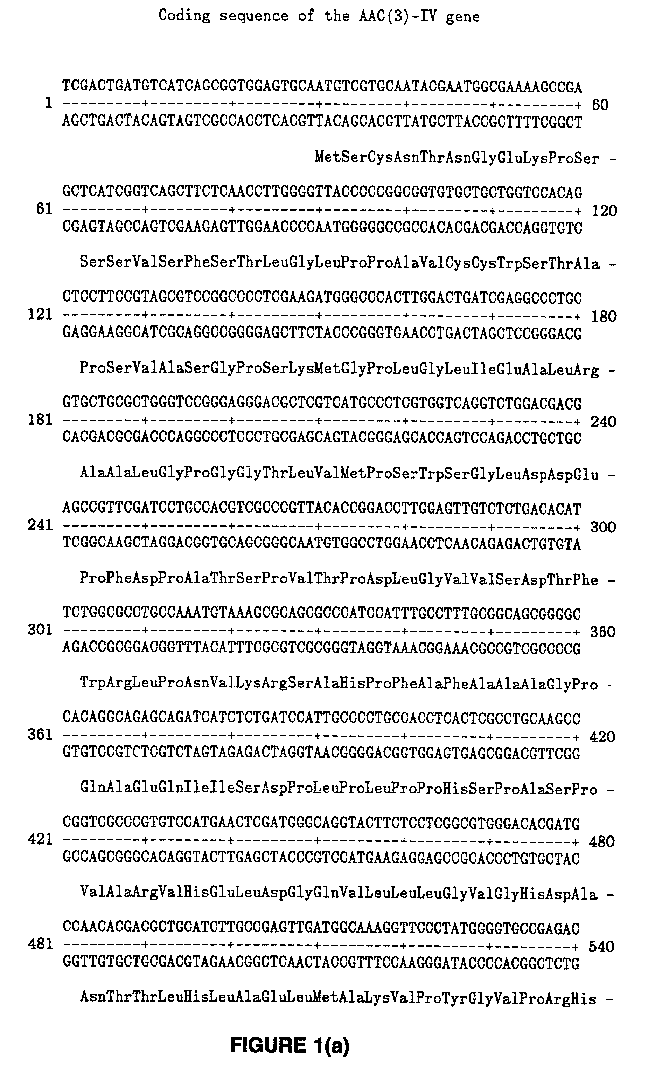

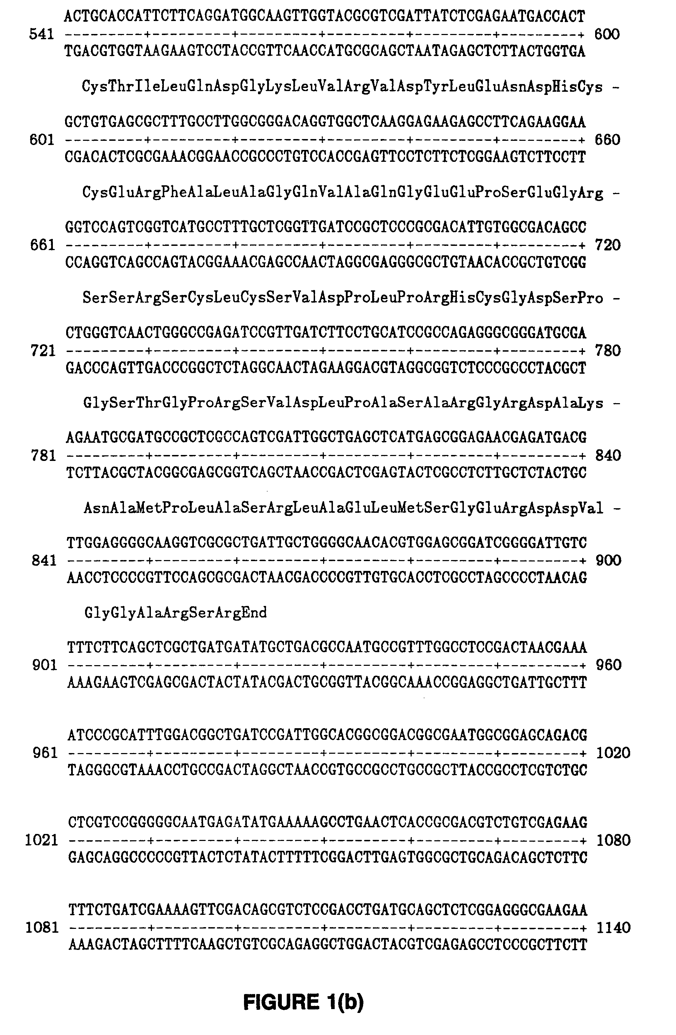

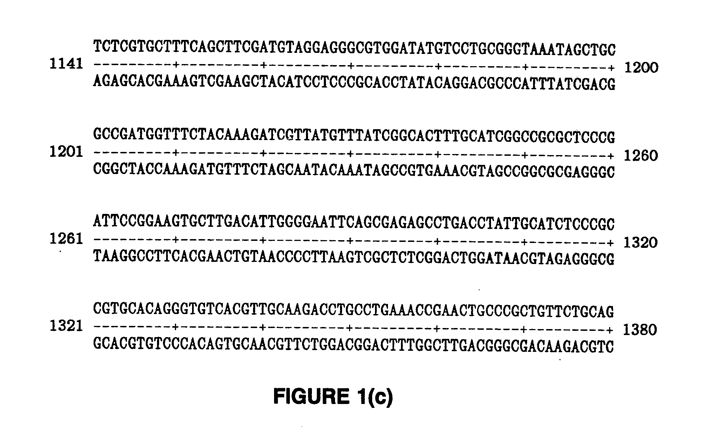

The DNA coding sequence for an AAC(3)-IV enzyme was excised from plasmid pLG62 (Gritz and Davies, 1984). The DNA sequence of this AAC(3)-IV enzyme has been reported in the literature (Brau et al., 1984) and is shown in Figure 1. Plasmid pMON825 which comprises the DNA containing the open reading frame (ORF) of this gene driven by the 35S promoter of cauliflower mosaic virus (CaMV35S) and the NOS 3′ polyadenylation signal was constructed in the following manner.

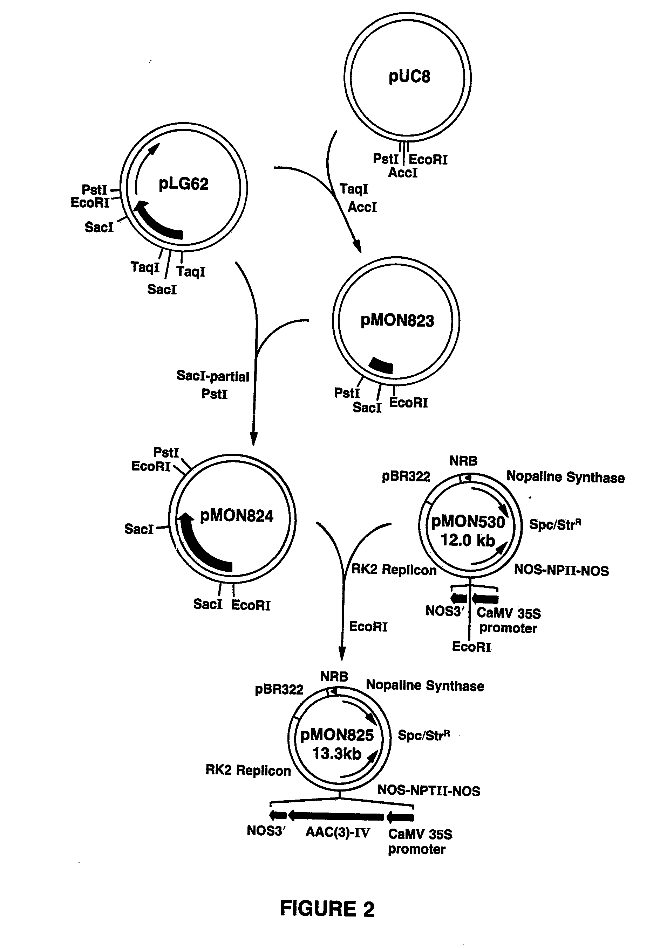

Referring to Figure 2, a 143 base pair (bp) TaqI fragment spanning the amino terminal portion of the ORF of the AAC(3)-IV gene was excised from pLG62 and cloned into the AccI site of plasmid pUC8 (Messing and Vieiva, 1982) creating pMON823. Next, a 1316 bp SacI-PstI fragment from pLG62 containing the remainder of the ORF was cloned into pMON823 previously cut with SaCI and PstI endonuclease. This plasmid was designated pMON824. Plasmid pMON824 contains the reconstructed coding sequence of the type IV gentamicin-3-N-acetyltransferase with an EcoRI site immediately upstream of the start of the ORF. A 1300 bp EcoRI fragment containing the entire ORF was then excised from pMON824 and cloned into the EcoRI site of pMON530. This plasmid was designated pMON825. Plasmid pMON825 contains the entire AAC(3)-IV ORF immediately downstream of the CaMV35S promoter and immediately upstream of the NOS 3′ transcriptional terminator/polyadenylation signal.

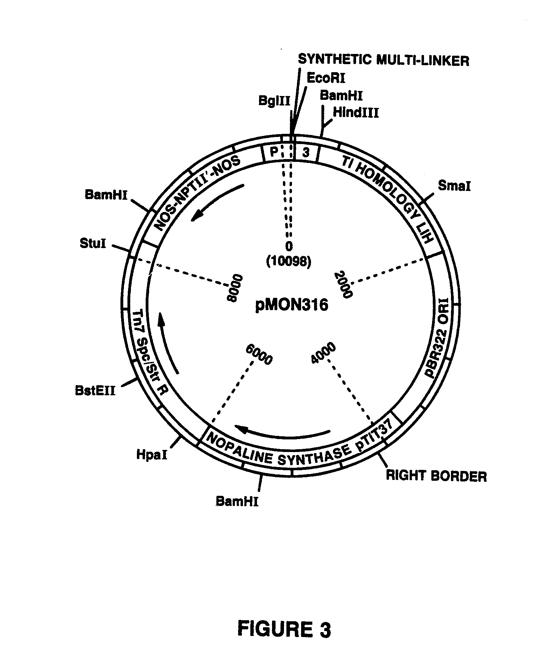

Plasmid pMON530 into which the AAC(3)-IV coding sequence was inserted is a binary vector for *Agrobacterium*-based plant transformation. The CaMV35S promoter was isolated from the pOS-1 clone of CM4-184 as an AluI (n 7143)-EcoRI* (n 7517) fragment which was inserted into BamHI cleaved pBR322, treated with the Klenow fragment of DNA polymerase I and then cleaved with EcoRI. The promoter fragment was then excised from pBR322 with BamHI and EcoRI, treated with Klenow polymerase and inserted into the SmaI site of M13mp8 so that the EcoRI site of the mp8 multi-linker was at the 5′ end of the promoter fragment. The nucleotide numbers refer to the sequence of CM1841 (Gardner et al., 1981). Site directed mutagenesis was then used to introduce a G at nucleotide 7464 to create a BglII site. The CaMV35S promoter fragment was then excised from the M13 as a 330 bp EcoRI-BglII fragment which contains the CaMV35S promoter, transcription initiation site and 30 nucleotides of the 5′ non-translated leader but does not contain any of the CaMV translational initiators nor the CaMV35S transcript polyadenylation signal that is located 180 nucleotides downstream from the start of transcription (Covey et al., 1981; Guilley et al., 1982). The CaMV35S promoter fragment was joined to a synthetic multi-linker and the NOS 3′ non-translated region and inserted into pMON200 (Fraley et al., 1985; Rogers et al., 1986) to give pMON316, see Figure 3.

Briefly, preparation of plasmid pMON200 begins with the EcoRI site of the synthetic multilinker which has the sequence:

5′-GAATTCATCGATATCTAGATCTCGAGCTCGCGAAAGCTT-3′

The multilinker ends with a HindIII site. The next fragment is the LIH or Left Inside Homology segment derived from pTiA6. This sequence is the reverse of bp 1618 to 3395 of the pTi15955 octopine-type T-DNA (Barker, et al., 1983). This segment begins with a HindIII site and ends with a BglII site that was made flush ended by treatment with Klenow polymerase and the four nucleotide triphosphates. This fragment was ligated to a fragment of pBR322 (Sutcliffe, 1978) from the PvuII site (bp 2069) to the PvuI site (bp 3740). This PvuI site was joined to a PvuI site located in the pTiT37 Ti plasmid approximately 150 bp from the end of the published sequence. Then bp 1 to bp 2102 of the nopaline T-DNA right border flanking sequence, the right border and entire nopaline synthase gene ending at a ClaI site were added (Depicker, 1982). This segment was joined to the Tn7 dihydrofolate reductase sequence (Fling, et al, 1983) beginning at the ClaI site at bp 560 and ending at the end of the published sequence at bp 883. The next segment of sequence comes from the Tn7 spectinomycin/streptomycin resistance determinant (Fling, et al., 1985) starting at bp 1 and ending after the second T of the EcoRI site at bp 1614. This EcoRI site was treated with Klenow polymerase and the four nucleotide triphosphates and joined to a similarly treated EcoRI site at the end of a synthetic linker joined to the 3′ end of the NOS 3′ non-translated region. The resultant sequence is

5′-GAATTAATTCCCGATCGATC-3′

The ATCGAT is the ClaI site located at bp 2102 of the nopaline synthase sequence (Depicker et al., 1982). The NOS 3′ sequence ends at the Sau3A site at bp 1847 of the nopaline synthase sequence and adjoins the following linker sequence:

5′-GGGGATCCGGGGG-3′

The last three G's of this sequence are from one half of the SmaI site located at bp 1118 of the Tn5 sequence (Beck et al., 1982). The Tn5 neomycin phosphotransferase II (NPTII) segment extends from the SmaI site at bp 1118 to the Sau3A site at bp 140. This Sau3A site is immediately adjacent to the following linker

5′-GTCTAGGATCTGCAG-3′

The T of the PstI site (CTGCAG) is the 3′ end of the nopaline synthase promoter segment which begins at bp 584 and ends with the BclI site at bp 284 of the nopaline synthase sequence (Depicker et al., 1982). The BclI site was cleaved with Sau3A and joined to a linker to give the following sequence which includes the half BclI site and the EcoRI which is the origin of the pMON200 plasmid

5′-TGATCCGGGGAATTC-3′

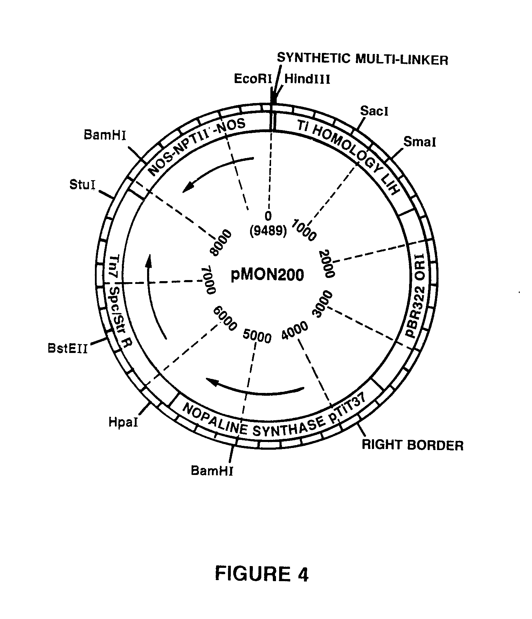

When assembled following the above instructions, the total size of the complete pMON200 (see Figure 4) plasmid will be 9489 bp.

Table I gives the endpoints of the various segments comprising pMON200 as well as the coordinates of the cleavage sites for the six base and larger recognition site restriction endonucleases. A list of endonucleases that do not cleave the vector is also included.

## Table I

## Major Regions and Restriction Endonuclease
## Cleavage Sites of pMON200

| Segment Name | Coordinates |
|---|---|
| Synthetic Multilinker | 1-36 |
| pTiA6 Fragment LIH | 36-1700 |
| pBR322 Origin Fragment | 1700-3476 |
| Right Border - Start | 3842 |
| pTiT37 Fragment | 3485-5730 |
| Nopaline Synthase Coding Region | 4217-5455 |
| Tn7 Spc/StrR Fragment | 5731-7911 |
| Spc/StrR Coding Region | 6390-7144 |
| NOS-NPTII'-NOS Kanamycin$^R$ Fragment | 7934-9489 |
| NPTII' Coding Region | 8366-9159 |

### Endonuclease Cleavage Site

| Endonuclease | Cleavage Site |
|---|---|
| AccI | 739, 1990 |
| AflII | 5509, 8154 |
| AsuII | 8352 |
| AvaI | 21, 1214, 3745 |
| BalI | 7694, 8954 |
| BamHI | 5028, 8191 |
| BbeI | 9037 |
| BclId | 3917, 6587 |
| BglI | 3231 |
| BglII | 17 |
| BstEII | 6549 |
| BvuI | 28, 822, 8675 |
| ClaI | 8 |
| ClaId | 510, 4389, 5731, 7934 |
| CvnI | 4100, 9300 |
| DraI | 629, 702, 749, 2977, 2996, 3814, 3891 |
| DraIII | 6526, 7143 |
| EcoRI | 1 |
| EcoRV | 12, 723 |
| FspI | 3333, 8934 |
| HindII | 4547, 4715, 5883 |
| HindIII | 34 |

## Endonuclease and Cleavage Site
### Continued

| | |
|---|---|
| HpaI | 5883 |
| MstI | 3333, 8934 |
| NaeI | 7040, 7551, 8251, 8534 |
| NarI | 9034 |
| NcoI | 504, 4607, 8600 |
| NdeI | 1252, 2041, 5978 |
| NheI | 523, 4115, 9284 |
| NruI | 30, 4768 |
| PstI | 3356, 8987, 9190 |
| PvuI | 3481, 6770, 7934, 8187, 9175 |
| PvuII | 367, 594, 4637, 7897, 8930 |
| RsrII | 8517 |
| SacI | 28, 822 |
| SacII | 4033, 9372 |
| SmaI | 1216 |
| SnaBI | 1023 |
| SpeI | 7809 |
| SphI | 191, 3771, 5212, 5316, 8635 |
| StuI | 7776 |
| TthI | 1965, 8917 |
| XbaId | 14 |
| XhoI | 21 |
| XmaIII | 9126 |
| XmnI | 617, 6034, 7133, 7924 |

d - indicates that the cleavage site is protected by dam methylation.

### Enzymes that do not cut

| | | | | | |
|---|---|---|---|---|---|
| AatII | ApaI | AvrII | BstXI | DraII | KpnI |
| MluI | NotI | PpuMI | SalI | ScaI | SfiI |

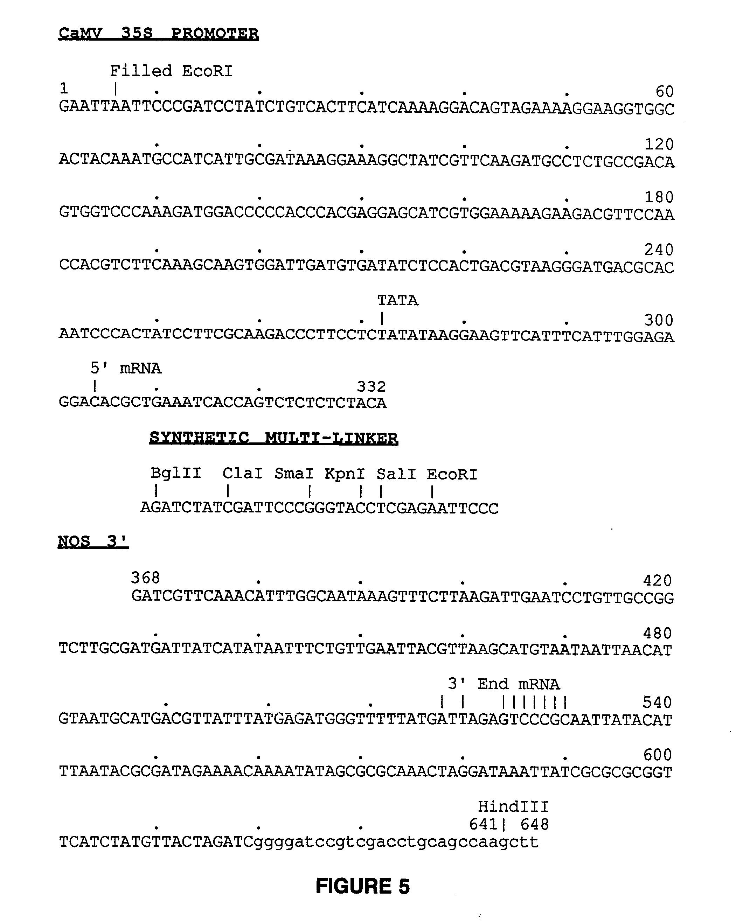

Co-integrating vector pMON316 contains unique cleavage sites for BglII, ClaI, KpnI, XhoI and EcoRI located between the 5′ leader and the NOS polyadenylation signals. Plasmid pMON316 retains all of the properties of pMON200. The complete sequence of the CaMV35S promoter, multi-linker and NOS 3′ segment is given in Figure 5. This sequence begins with the XmnI site created by Klenow polymerase treatment to remove the EcoRI site located at the 5′ end of the CaMV35S promoter segment.

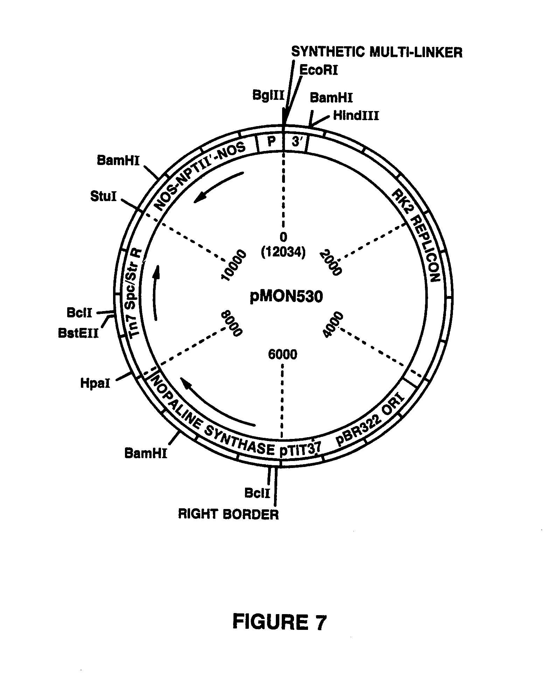

Plasmid pMON530 (see Figure 7) is a derivative of pMON505 prepared by transferring the 2.3 kb StuI-HindIII fragment of pMON316 into pMON526. Plasmid pMON526 is a simple derivative of pMON505 in which the SmaI site is removed by digestion with XmaI, treatment with Klenow polymerase and ligation. Plasmid pMON530 retains all the properties of pMON505 and the CaMV35S-NOS expression cassette and now contains a unique cleavage site for SmaI between the promoter and polyadenylation signal.

Binary vector pMON505 is a derivative of pMON200 in which the Ti plasmid homology region, LIH, has been replaced with a 3.8 kb HindIII to SmaI segment of the mini RK2 plasmid, pTJS75 (Schmidhauser &; Helinski, 1985). This segment contains the RK2 origin of replication, oriV, and the origin of transfer, oriT, for conjugation into *Agrobacterium* using the tri-parental mating procedure (Horsch Klee, 1986).

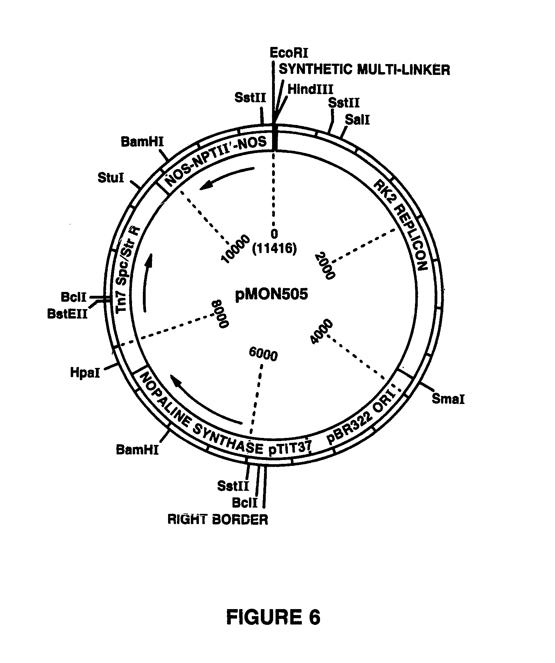

Referring to Figure 6, plasmid pMON505 retains all the important features of pMON200 including the synthetic multi-linker for insertion desired DNA fragments, the chimeric NOS/NPTII′/NOS gene for kanamycin resistance in plant cells, the spectinomycin/streptomycin resistance determinant for selection in *E. coli and A. tumefaciens*, an intact nopaline synthase gene for facile scoring of transformants and inheritance in progeny and a pBR322 origin of replication for ease in making large amounts of the vector in *E. coli*. Plasmid pMON505 contains a single T-DNA border derived from the right end of the pTiT37 nopaline-type T-DNA. Southern analyses have shown that plasmid pMON505 and any DNA that it carries are integrated into the plant genome, that is, the entire plasmid is the T-DNA that is inserted into the plant genome. One end of the integrated DNA is located between the right border sequence and the nopaline synthase gene and the other end is between the border sequence and the pBR322 sequences.

Plasmid pMON825 was mobilized into *Agrobacterium tumefaciens* strain A208 carrying the disarmed Ti plasmid, pTiT37-SE. This disarmed nopaline-type Ti plasmid was created from pTiT37 in a manner analogous to that described by Fraley et al. (1985) for creating the pTiB6S3-SE disarmed octopine-type Ti plasmid. The general procedure is to replace most of the pTiT37 T-DNA with a selectable marker and pBR322 and LIH segments from pMON200 to provide a region of homology for recombination with pMON200 and derivatives thereof. This replacement results in the deletion of the rightmost approximately 90 percent of the T-DNA including the phytohormone biosynthetic genes, nopaline synthase gene and the right border of the T-DNA.

The source of the pTiT37 sequences was the plasmid MINI-Ti described by deFramond et al., (1983). This plasmid is a convenient source; however, these same Ti plasmid segments could be obtained directly from the pTiT37 or related pTiC58 plasmid or from subclones of these plasmids isolated by others such as those described by Hepburn et al., (1983) or Zahm et al., (1984).

Plasmid MINI-Ti is a derivative of pBR325 carrying the pTiT37 KpnI fragments 13b, 4 and 11 (deFramond et al., 1983) which are analogous to the pTiC58 KpnI fragments 13, 3 and 12 (Depicker et al., 1980). The internal T-DNA sequences including the phytohormone biosynthetic genes and right border were removed from mini-Ti by digestion with HindIII and religation to produce pMON284. The pMON284 plasmid contains a unique KpnI site which was converted to a BamHI site by cleavage with KpnI and insertion of the following synthetic linker:

    5'- CGGATCCGGTAC -3'
    3'- CATGGCCTAGGC -5'

which contains a BamHI site (5'-GGATCC) in the the center of the linker. A plasmid that contains this linker was isolated and called pMON293.

The pMON293 plasmid carries the following pTiT37 fragments adjacent to each other in inverted orientation with respect to their orientation in the Ti plasmid and joined through a BamHI linker. First is the KpnI site at the right end of the 13b fragment. This fragment contains the left border of the pTiT37 T-DNA. The left end of the 13b fragment is joined to the BamHI linker. Joined to this is the right end of the KpnI 11 fragment. This fragment contains Ti plasmid located to the right of the T-DNA and ends with a HindIII site that is the right end of the pTiC58 HindIII 2 fragment (Depicker et al., 1980). This is joined to the pBR325 derivative plasmid which also is fused to the KpnI site at the right end of the KpnI 13b fragment.

To introduce homology to pMON200 and a kanamycin resistance selectable marker for *A. tumefaciens* between the pTiT37 segments plasmid pMON292 was constructed. Plasmid pMON292 is a derivative of pMON113 which consists of the 2.6 kb pBR322 PvuII to HindIII fragment joined to the 1.7 kb BglII (nucleotide 1617) to HindIII (nucleotide 3390, Barker et al., 1983) fragment of octopine type T-DNA of pTiA6. This segment, called the LIH, has been previously described by Fraley et al. (1985). The BglII site was made flush ended by treatment with Klenow polymerase and the four nucleotide triphosphates before ligation with the pBR322 segment.

Plasmid pMON113 was cleaved with HindIII, treated with Klenow polymerase and joined to the 1.2 kb AvaII fragment of Tn903 (Oka et al., 1981) that had been treated with Klenow polymerase, ligated to synthetic BamHI linkers, digested with BamHI and treated again with Klenow polymerase. The resulting plasmid carrying the Tn903 kanamycin resistance determinant adjacent to the LIH segment was called pMON292.

The pMON200 homology region and bacterial kanamycin resistance marker were inserted between the pTiT37 segments by mixing pMON292 linearized by cleavage with HincII with two fragments derived from pMON293: a 2.5 kb PvuII-BamHI fragment and a 4.5 kb fragment isolated after cleavage with HindIII, Klenow polymerase treatment, and cleavage with BamHI. The resulting plasmid, pMON313, carries the following fragments in this order. First, is the BamHI linker followed by a 4.5 kb KpnI-HindIII fragment derived from the right side of pTiT37 KpnI fragment 11. This is joined to the 750 bp HincII-HindIII segment of pBR322 followed by the 1.2 kb Tn903 segment encoding kanamycin resistance. This is followed by the LIH (HindIII segment and the PvuII-HincII segment of pBR322 that carries the origin of replication). Next, there is a 2.5 kb PvuII to KpnI fragment from the left end of the pTiT37 KpnI 13b fragment which contains the left border of the T-DNA. Finally, this is joined to the starting BamHI linker.

To introduce this DNA into Agrobacterium, pMON313 was cleaved with BamHI and mixed with pRK290 DNA that had been cleaved with BglII and treated with DNA ligase. A derivative of pRK290 carrying the pMON313 plasmid was isolated and called pMON318.

Plasmid pMON318 was introduced into *Agrobacterium tumefaciens* strain A208 which carries pTiT37 and a chromosomal chloramphenicol resistance by standard bacterial mating methods using pRK2013 as a helper. This method and subsequent selection for the replacement of the T-DNA with the engineered T-DNA segment carried in pMON318 was done as described by Fraley et al. (1985) for the selection of the disarmed octopine-type pTiB6S3-SE plasmid.

The resultant disarmed pTiT37-SE plasmid contains the vir region intact and retains the left T-DNA border and approximately 1 kb of the T-DNA. This region of the T-DNA has not been reported to encode a transcript (Joos et al., 1983). This is followed by the pBR322 segment of LIH and then the Tn903 kanamycin resistance. The Tn903 segment is joined to a 750 bp segment of pBR322 that is joined to the left end of the pTiT37 analogue of the pTiC58 HindIII 2 fragment (Depicker et al., 1980). This fragment is located outside the right end of the pTiT37 T-DNA. The result is that over 90% of the T-DNA including the phytohormone biosynthetic genes responsible for crown gall disease production and right border are absent from the pTiT37-SE plasmid.

Mating was done by the tri-parental conjugation system using the helper plasmid pRK2013 (Ditta et al., 1980). Transconjugants, designated pMON825-ASE, were selected on LB plates (Miller, 1972) containing 50μg/ml kanamycin, 25 μg/ml chloramphenicol and 50 μg/ml spectinomycin. The resulting cells were used to transform petunia cells.

The basic leaf disc transformation system has been described (Horsch et al., 1985). Leaf discs of *Petunia hybrida* VR (F1 Violet 23 x Red 51) were precultured for 2 days on MS104 medium [MS salts (Gibco), B5 vitamins, sucrose (30 g/l), benzyladenine (1.0 μg/ml), napthaline acetic acid (0.1 μg/ml), and 0.8% agar]. The discs were then soaked for a few minutes in an overnight culture of pMON825-ASE, blotted dry, and placed upside-down on MS104 nurse culture plates (Horsch et al., 1980). After three days of coculture, the discs were transferred to MS104-500 μg/ml carbenicillin plates containing levels of gentamicin (Sigma, St. Louis) ranging between 30 μg/ml and 300 μg/ml of medium for selection of transformed callus.

Control tissue transformed with plasmid pMON530 was also grown on gentamicin. While growth of control tissue was completely inhibited by 100 μg/ml gentamicin, the growth of pMON825 transformed tissue was uninhibited by the antibiotic. These results clearly demonstrate that the chimeric AAC(3)-IV gene confers a selectable phenotype on transformed plant cells.

Example 2

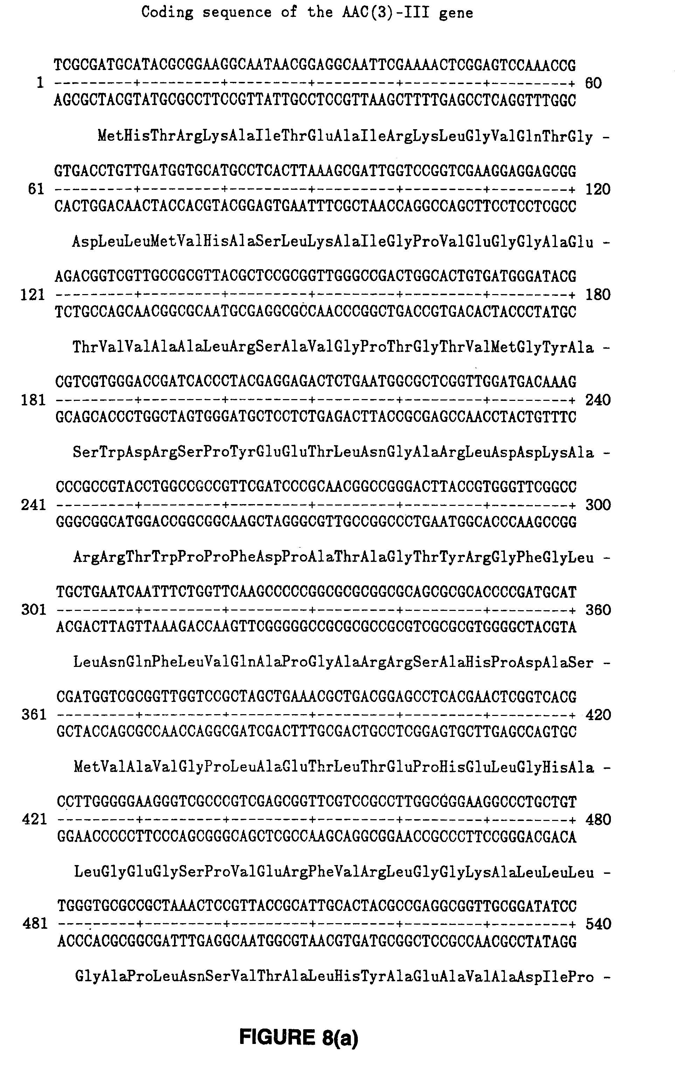

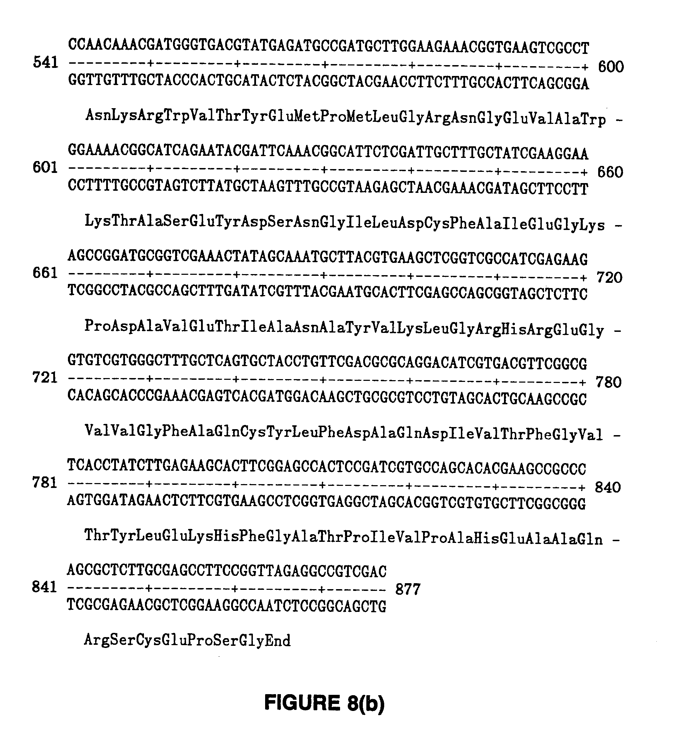

The DNA coding sequence for an AAC(3)-III enzyme was excised from plasmid pWP866 (Allmansberger et al., 1985) on a 869 bp fragment using endonucleases NruI and SalI. The DNA sequence of this AAC(3)-III enzyme is shown in Figure 8.

Plant transformation vector pMON847 was prepared by cloning the 869 bp NruI/SalI fragment into SmaI/XhoI cleaved pMON530 (Figure 7). The resulting binary vector pMON847 contains the NOS/NPTII/NOS gene as well as the chimeric CaMV35S/AAC(3)-III/NOS gene.

Plasmid pMON847 was mobilized into *Agrobacterium tumefaciens* strain A208 carrying the disarmed Ti plasmid, pTiT37-SE. Mating was done by the triparental conjugation system using helper plasmid pRK2013 (Ditta, et al., 1980). Transconjugants (pMON847-ASE) were selected on LB plates (Miller, 1972) containing 50 μg/ml kanamycin, 25 μg/ml chloramphenicol and 50 μg/ml spectinomycin. The resulting cells were used to transform petunia cells as described above in Example 1.

Leaf discs of petunia transformed with pMON505 were used as controls. Transformed tissue was selected on 100 μg/ml and 300 μg/ml gentamicin. Tissue transformed with pMON847 produced gentamicin tolerant callus at both levels of selection while the growth of control tissue was completely inhibited by 100 μg/ml gentamicin. Initial studies indicate pMON847 transformed petunia tissue forms more callus on 300 μg/ml gentamicin than does tissue transformed with pMON825.

Example 3

The transformation vectors of Example 1 and 2 were used for immediate selection for transformed soybean callus as described below.

Soybean seedlings (*G. max*, cv. Peking) were germinated for 5 days on sterile 0.8% agar (aq.). Hypocotyls were excised aseptically and cut into segments 5 mm long. Cut ends of each hypocotyl segment were smeared with the appropriate *Agrobacterium* strain. The hypocotyls were co-cultured with the *Agrobacterium* strain by placement directly on agar solidified 1/10 SH salts (Schenk et al., 1972) plus 3% sucrose for 2-3 days. Both armed and disarmed *Agrobacterium* were used to insert pMON825 and pMON847 in soybean hypocotyls. The armed *Agrobacterium* used was strain A208 carrying plasmid pTiT37. The disarmed *Agrobacterium* used was strain ASE carrying disarmed plasmid pTiT37-SE (described hereinbefore).

After co-culture, the hypocotyl segments inoculated with the disarmed strains were transferred to agar solidified MS medium (Murashige and Skoog, 1962) containing 4.68 mg/l NAA, 2.5 mg/l kinetin and 500 mg/l carbenicillin. Hypocotyls inoculated with armed strains were placed on MS medium but without the plant growth regulators NAA and Kinetin. Observations as to callusing ability of the explants were made at four weeks. Opine assays were performed on callus at 4-6 weeks.

Referring to Table II below, soybean hypocotyls which had been inoculated with ASE::pMON825 (containing the AAC(3)-IV gene) were able to produce callus on 50, 100 or 250 mg/l gentamicin. Control hypocotyls which had been inoculated with ASE::pMON505 or ASE::pMON200 produced no callus at the same gentamicin concentrations. Hypocotyls which had been inoculated with A208::pMON505 or A208::pMON200 were capable of callusing only on the lowest gentamicin concentration indicating that the armed vectors allowed escape callusing on 50 mg/l gentamicin. The binary A208::pMON825 vector was most effective in producing transformed soybean tissue.

### Table II
#### Hypocotyl Segments which produce Callus

| Construct | Sample Size | Gentamicin (ml/1) | | | |
|---|---|---|---|---|---|
| | | 0 | 50 | 100 | 250 |
| ASE/505 | 30 | 30 | 0 | 0 | 0 |
| ASE/200 | 30 | 30 | 0 | 0 | 0 |
| ASE/825 | 30 | 27 | 0 | 2 | 0 |
| A208/505 | 30 | 30 | 6 | 0 | 0 |
| A208/200 | 30 | 30 | 5 | 0 | 0 |
| A208/825 | 30 | 18 | 18 | 12 | 5 |

Referring to Table III below, a second gentamicin marker construct, pMON847, containing the AAC(3)-III gene was used to immediately select transformed soybean hypocotyls as previously described. The data of Table III demonstrate that the AAC(3)-III gene of pMON847 functions in the immediate selection of transformed, gentamicin resistant callus.

### Table III
#### Hypocotyls Segments which produce Callus

| Replication # | Sample Size | ASE/847 | | A208/847 | |
|---|---|---|---|---|---|
| | | 0 Gent | 100 Gent | 0 Gent | 100 Gent |
| 1 | 20 | 20 | 7 | 20 | 3 |
| 2 | 20 | 20 | 7 | 20 | 7 |
| 3 | 20 | 20 | 6 | 20 | 11 |
| 4 | 20 | 20 | 6 | 20 | 6 |
| 5 | 20 | 20 | 1 | 20 | 7 |
| 6 | 20 | 20 | 2 | 20 | 12 |

The soybean callus produced under gentamicin selection usually emerged as small sectors from the cut ends of the hypocotyl segments. The calli usually ranged in color from brown to green. The gentamicin selected calli were strongly opine positive regardless of their color. However, it was primarily the green calli that were capable of continued growth on 250 mg/l gentamicin, although about one-third of the brown calli produced new greenish callus after subculture on gentamicin. The production of green callus under initial gentamicin selection is considered important for full recovery of gentamicin resistant tissue.

Example 4

The transformation vector of Example 2 containing the chimeric AAC(3)-111 gene was used for immediate selection of transformed *Brassica napus* (Canola, rape oil seed) as described below.

Stem segments of *Brassica napus* which were inoculated and co-cultured with a disarmed *Agrobacterium* strain containing the AAC(3)-III marker gene (ASE::pMON847) produced shoots 3-6 weeks after transfer to selection medium which contained 100 µg/ml gentamicin. Control stem segments inoculated with the corresponding *Agrobacterium* strain which did not contain the chimeric gentamicin resistance gene did not form shoots on 100 µg/ml gentamicin medium. Transformants were identified by the ability of their leaf tissue to form callus on media containing 100 µg/ml gentamicin while wild-type *B. napus* tissue dies. The production of nopaline synthase in the progeny of the *Brassica napus* plant transformed and regenerated by the stem segment procedure described above demon strates the efficacy of the system for the production of transgenic plants. Seeds from the transgenic plant transformed with pMON847 and from a wild-type plant were germinated on media with 0 and 100 µg/ml gentamicin. The nopaline positive seedlings produced very green cotyledons on gentamicin while the control cotyledons bleached on this level gentamicin. Seedlings from both the wild-type and transgenic plant produced green healthy seedings on the media without gentamicin. The gentamicin tolerance and production of nopaline synthase in the progeny are important results because they are definitive proof that the *Brassica napus* stem segments transformation/regeneration procedure produces transgenic plants.

The 100 μg/ml gentamicin selection used in the above-identified *Brassica napus* transformation/regeneration protocol produced a high number of transformed plants. Unfortunately, the protocol also allowed a relatively high number of escapes (growth of untransformed tissue). Selection of transformed *Brassica napus* shoots at 100, 200 and 300 μg/ml gentamicin were compared to determine if higher gentamicin levels would tighten the selection of transgenic shoots. Initial experiments indicate that selection at 200 μg/ml and 300 μg/ml gentamicin is too high since no transformed shoots were produced even though one escape came through at 200 μg/ml gentamicin. Levels of gentamicin between 100 and 200 μg/ml may result in reduced occurrence of escape while permitting the production of a high number of transformed plants.

Example 5

The above-described plant transformation vectors pMON825 and pMON847 demonstrate the utility of the gentamicin marker genes for plant transformation. However, pMON825 is not a particularly practical cloning vector because it contains many restriction sites in the chimeric CaMV35S/AAC(3)-IV/NOS construct. To prepare a more useful plant transformation vector containing only the chimeric AAC(3)-IV gene as the selectable marker, restriction sites had to be deleted from pMON825 and the chimeric AAC(3)-IV gene introduced into other constructs as described below. While the preparation of a binary *Agrobacterium*-based plant transformation vector is described below, it should be understood that the gentamicin marker gene of the present invention can also be utilized with co-integrating vectors such as pMON316. Such vectors can be easily prepared following the general teachings described herein.

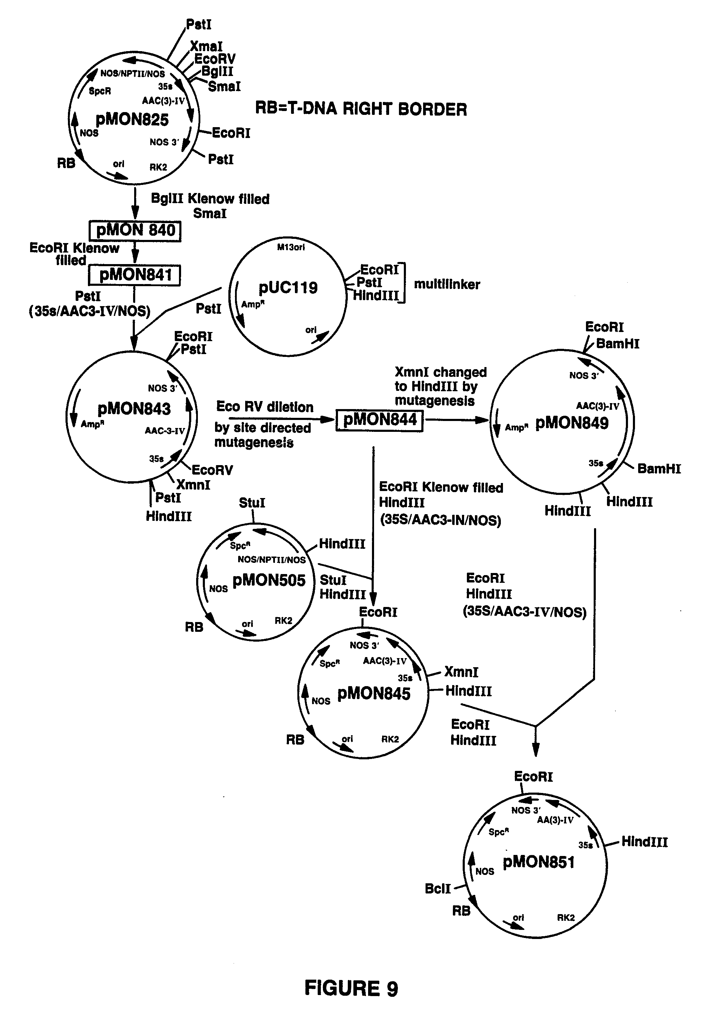

Referring to Figure 9, plasmid pMON825 was cut with endonucleases SmaI and BglII. The overhangs resulting from the BglII cut were filled by treatment with Klenow polymerase and the four nucleotide triphosphates. The flush ends were ligated by treatment with DNA ligase and the resulting plasmid designated pMON840. Plasmid pMON840 was cut with endonuclease EcoRI. The overhangs were filled by treatment with Klenow polymerase and the four nucleotide triphosphates. The flush ends were ligated by treatment with DNA ligase and the resulting plasmid designated pMON841. The above procedures removed the BlgII, SmaI and EcoRI restriction sites, as well as other extraneous restriction sites between the BglII and SmaI sites, from the chimeric CaMV35S/AAC(3)-IV/NOS gene.

Other restriction sites were removed by site directed mutagenesis in the following manner. The 2170 bp PstI fragment of pMON841 was introduced into PstI cut pUC119 producing a construct designated pMON843. pUC119 is constructed by isolating the 476 bp HgiAI/DraI fragment of bacteriophate M13 and making the ends blunt with T4 DNA polymerase (New England Biolabs). This fragment is then inserted into pUC19 that had been digested with NdeI and filled with Klenow DNA polymerase (New England Biolabs).

The EcoRV site in the CaMV35S promoter sequence was deleted by site directed mutagenesis (Zoller, 1983) using the oligonucleotide.

5'-TTACGTCAGTGGAAGTATCACATCAATCCA-3'

producing plasmid pMON844. Sequencing confirmed that pMON844 contained the correct mutation.

The NOS/NPTII/NOS gene in pMON505 was removed by cleavage of pMON505 with StuI and HindIII. It was replaced with the 2220 bp EcoRI (Klenow filled)/HindIII fragment from pMON844 containing the CaMV35S/AAC(3)-IV/NOS gene. This plasmid was designated pMON845. It was subsequently determined that the CaMV35S/AAC(3)-IV/NOS chimeric gene which is in pMON845 contained extraneous sequence upstream from the start of the CaMV35S promoter sequence carried from the NOS promoter of pMON825. The XmaI site at the start of the CaMV35S promoter of pMON844 was changed to a HindIII by site directed mutagenesis using the oligonucleotide.

5'-TGTAGGATCGGGAAGCTTCCCCGGATCATG-3'

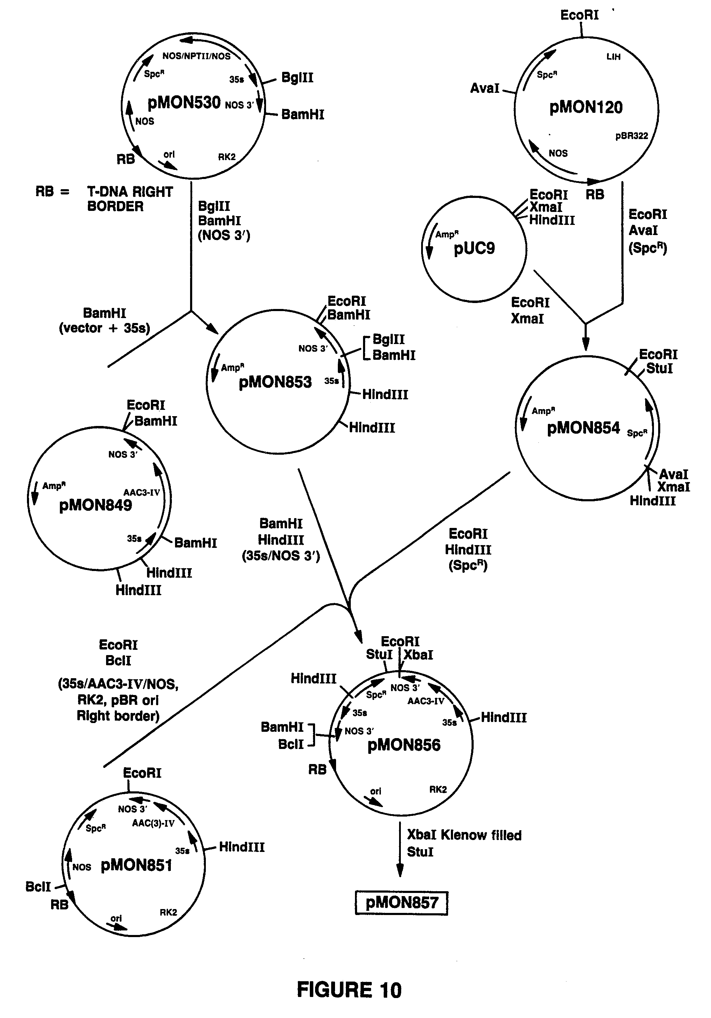

producing plasmid pMON849. The EcoRI/HindIII fragment of pMON845 (containing the CaMV35S/AAC(3)-IV/NOS gene) was replaced with the smaller 1900 bp EcoRI/HindIII fragment of pMON849 carrying the same chimeric gene. This plasmid was designated pMON851. Fragments were used from three plasmids to construct a multipurpose cloning vector employing the CaMV35S/AAC(3)-IV/NOS gene as a selectable marker and containing a CaMV35S/NOS3' cassette. Referring to Figure 10, the CaMV35S/NOS3' cassette was prepared from pMON849 and pMON530. Specifically, pMON849 was cut with BamHI removing the AAC(3)-IV/NOS sequence while leaving the CaMV35S sequence. The 294 bp BglII/BamHI fragment from pMON530 containing the multilinker and NOS3' and was ligated into the BamHI cut pMON849 producing pMON853.

The Tn7 Spc/Str resistance gene was obtained from pMON120. The 1600 bp EcoRI/AraI fragment of pMON120 containing the Spc/Str gene was cloned into EcoRI and XmaI cut pUC9 producing plasmid pMON854.

Plasmid pMON856 was prepared by ligation of the following three fragments:
Fragment #1: The 7770 bp EcoRI to BclI fragment from pMON851 containing the CaMV35S/AAC(3)-IV/NOS selectable marker gene, RK2 replicon, pBR322 origin of replication, and the right border sequence from plasmid pTiT37 of *Agrobacterium tumefaciens*.

Fragment #2: The 630 bp HindIII to BamHI fragment from pMON853 containing the CaMV35S/NOS3' cassette and the multilinker.

Fragment #3: The 1630 bp EcoRI to HindIII fragment from pMON854 containing the Spc/str resistance gene.

Extraneous sequence and restriction sites between the selectable marker gene and the Spc/Str gene were removed from pMON856 in the following manner. Plasmid pMON856 was cut with StuI and XbaI. The XbaI site was filled by treatment with Klenow polymerase and the four nucleotide triphosphates.

Subsequent ligation produced plasmid pMON857 which is a useful plant transformation vector containing the CaMV35S/AAC(3)-IV/NOS selectable marker gene.

### Example 6

## TRANSFORMATION OF ALFALFA USING GENTAMICIN AS A SELECTABLE MARKER

### Plant Material

*Medicago sativa* L. cv. Regin S line RA≠3. RA≠3 is a line derived from one plant in the Regin S population developed by Dr. Ted Bingham, University of Wisconsin, Madison, WI which was identified as highly regenerable in tissue culture and is propagated by cuttings. Plants were grown in a growth chamber at a constant temperature of 21°C under a 14 hour photo-period. Plants were watered 4 times a day and fertilized weekly with Peters General Purpose Fertilizer.

### Bacterial Strains

Innoculations were made with *Agrobacterium tumefaciens* strain ASE, derived from A208 carrying the disarmed plasmid pTiT37SE. Intermediate plant transformation vectors pMON847 and pMON200 were mated into the strain, giving binary strains pMON847-ASE and pMON200-ASE, respectively. The pMON847-ASE and pMON200-ASE were grown in Luria broth for 2 days prior to innoculation. On the second day a fresh culture wa innoculated from the first culture.

### Sensitivity of Alfalfa Leaf Tissue to Gentamicin

The sensitivity of alfalfa cells to gentamicin was determined as follows. Alfalfa leaf tissue was exposed to gentamicin at concentrations ranging from 10 to 300 µg/ml. Fresh weights were taken at 4 weeks. At 30 µg/ml gentamicin there was a significant number of explants producing callus but 100 µg/ml gentamicin completely inhibited all callus formation. These results show that alfalfa leaf tissue is very sensitive to gentamicin resulting in inhibition of growth or death of all cells.

### Transformation/Selection/Regeneration

### Innoculation

Fully expanded, light green, healthy leaves were selected. The leaves were surface sterilized in 20% Clorox plus 4 drops Tween-20 per 100 mls for 6 minutes and rinsed 3 times with sterile water. Leaves were cut horizontally across the midrib into 3-4 sections in a sterile 15X100 mm petri plate. The leaf explants were innoculated for 5 minutes by submersion in a 5x10⁸ bacteria/ml culture of the *Agrobacterium* strains, and blotted dry with sterile filter paper.

The pMON847-ASE and pMON200-ASE innoculated leaf tissue was cultured basal side up on feeder plates on medium containing 1/10X Schenk and Hildebrant (SH) standard salts, SH vitamins, 3% sucrose, 0.8% agar, 25 µM Napthaline Acetic Acid (NAA), 10µM kinetin, pH 5.5 and 1.5 mls TXD feeder cells.

After a two day coculture period, leaf pieces were transferred to fresh SH medium containing standard SH salts, the same vitamins, sucrose and growth regulators as in the coculture medium, plus 500 µg/ml carbenicillin, 0.5 mM arginine, and gentamicin at 0, 50 and 100 µg/ml. At 3-4 weeks leaf tissue was cut up to eliminate necrotic tissue and healthy tissue transferred to fresh plates containing the same medium. Culture of explants was in a growth room under a 14 hour photoperiod and at 26°C constant temperature.

At 6 weeks much of the original explant and developing callus was necrotic but some of the pMON847-ASE innoculated callus was yellow to green and healthy on medium containing 50 and 100 µg/ml gentamicin. The pMON200-ASE innoculated control callus was brown and necrotic on 50 or 100 µg/ml gentamicin. Some of the gentamicin resistant pMON847-ASE innoculated callus was strongly opine positive at 6 weeks.

Twenty independent healthy yellow to green calli innoculated with pMON847-ASE and growing on gentamicin at 50 or 100 µg/ml were induced for 4 days on SH medium containing 50 µM 2,4-D, 5µM kinetin, 500 µg/ml carbenicillin and the same 50 or 100 µg/ml gentamicin that was used for selection. The induced calli was transferred to SH media with 50 mM proline, 500 µg/ml carbenicillin and the same gentamicin level for regeneration. Shoots were first observed at 3¹/₂ weeks. Plantlets which had regenerated from 3 transformation events after 2 months were transferred to soil and assayed for nopaline, gentamicin acetyltransferase activity, and gentamicin resistance in leaf tissue, see Table IV below.

## TABLE IV

| Transformation Event and Plant Number | NOP Assay | Gent. DOT BLOT Assay | 100 ug/ml Gent. Leaf Assay |
|---|---|---|---|
| O-1 | - | + | + |
| O-2 | - | + | + |
| O-3 | - | + | + |
| O-4 | - | +. | + |
| O-5 | - | + | + |
| O-6 | - | + | + |
| O-7 | - | + | + |
| O-8 | - | + | + |
| O-9 | - | + | + |
| D-1 | - | + | + |
| D-2 | - | + | + |
| D-3 | - | + | + |
| P-1 | - | + | + |
| P-2 | + | + | + |
| P-3 | - | + | + |
| P-4 | - | NT | + |
| P-5 | + | NT | + |
| P-6 | + | + | + |
| P-7 | +/- | NT | + |
| P-8 | + | - | + |
| P-9 | + | NT | + |
| P-10 | - | NT | + |
| Wild-type RA3 (Control) | - | - | - |

The letter designates the transformation event and the number designates the number of plants from that event.

NT = Not Tested

Five plants out of the 22 were nopaline positive and the nopaline positive plants were all produced from the same transformation event. Leaf tissue from each of the 22 plants formed callus on medium containing 100 µg/ml gentamicin while wild-type control tissue did not callus and is bleaching at 3 weeks. A dot blot assay for the gentamicin acetyltransferase enzyme was positive for 16 of 17 plants selected. The gentamicin dot blot assay, the nopaline positive plants and the gentamicin leaf assay results are all consistent with the production of transgenic plants. These results demonstrate the utility of gentamicin resistance genes as a selectable marker for producing transgenic alfalfa plants.

REFERENCES

Allmansberger, R., Brau, B., Piepersberg, W. (1985) Mol. Gen. Genet. 198: 514-520.
Bevan, M., et al. Nature 304:184 (1983).
Barker, R. F., Idler, K. B., Thompson, D. V. and Kemp, J. D., Plant Molec. Biol. 2, 335 (1983).
Beck, E., Ludwig, G., Auerswald, E. A., Reiss, B. and Schaller, H., Gene 19,327 (1982).
Brau et al., Mol Gen Genet 193:179-187 (1984).
Brzezinska, M., Benveniste, R., Davies, J., Daniels, P. J. L., Weinstein, J. (1972) Biochemistry 11:761:766.
Covey, S., Lomonosoff, G. and Hill, R., Nucleic Acids Res. 9, 6735 (1981).
Davies, J., O'Conner, S., Antimicrob. Agents Chemother. 14:69-72 (1978).
DeFramond, et al., Bio/Technology 1:262 (1983).
Depicker, A., Stachel, S., Dhaese, P., Zambryski, P and Goodman, H., Molec. Appl. Genetics 1, 561 (1982).
Depicker, et al., Plasmid 3:193-211 (1980).
Ditta, G., Stanfield, S., Corbin, D., Helinski, D. R., (1980) Proc. Natl. Acad. Sci. U.S.A. 77:7347-7351.

Fling, M. and Richards, C., Nucleic Acids Res. 11, 5147 (1983).

Fling, M., Kopf., J. and Richards, C., Nucleic Acids Res. 13, 7095 (1985).

Fraley, R., Rogers, S., Eichholtz, D., Flick, J., Fink, C., Hoffman, N. and Sanders, P., Bio/Technology 3, 629 (1985).

Gardner, R., Howarth, A., Hahn, P., Brown-Luedi, M., Shepherd, R. and Messing, J., Nucleic Acids Res. 9, 2871 (1981).

Guilley, H., Dudley, R., Jonard, G., Balax, E. and Richards, K., Cell 30, 763 (1982).

Gritz and Davies, Gene 25:179-188 (1984).

Hepurn, et al., J. Mol. Appl. Genetics 2:211-224 (1983).

Herrera-Estrella, L., et al. Nature 303:209 (1983).

Horsch, R. and Klee, H., Proc. Natl. Acad. Sci. U.S.A Vol 83, 4428-4432 (1986).

Horsch, R., Fry, J., Hoffmann, N., Wallroth, M., Eichholtz, D., Rogers, S., Fraley, R. Science 227:1229-1231 (1985).

Horsch, R. and Jones, G. In Vitro 16:103-108 (1980).

Joos, et al., Cell 32:1057-1067 (1983).

Klee, H. J. et al., Bio/Technology 3:637-642 (1985).

LeGoffic, F., Martel, A., Witchitz, J., (1974) Antimicrob. Agents Chemother. 6:680-684.

Messing, J. and Vieira, J. (1982) Gene 19:269-276.

Murashige, T. and Shoog, F., (1962) Physiol Plant 15:473.

Miller, J. H. (1972) Nucl Acids Res. 7:1513-1523.

Oka, et al., J. Mol. Biol. 147:217 (1981).

Rogers, S., Horsch, R. and Fraley, R., Methods in Enzymology Vol 118, (H. Weissbach and A. Weissbach, eds). p. 627 Academic Press, New York (1986).

Rosenthal, S., Davies, J., Freundlich, L., Hoffman, B., (1976) 16th ICAAC Meeting, No. 205 (Abstract).

Schank, R. V., Hildebrandt, A. C. (1972) Can J. Bot. 50:199-204.

Schmidhauser, T. and Helinski, D., J. Bacteriology, 164, 446 (1985).

Sutcliffe, J. G., Proc. Natl. Acad. Sci. USA 75, 3737 (1978).

Umezawa, H., Yagisawa, M., Matsuhashi, Y, Naganawa, H., Yamamoto, H., Kondon, S., Takcuchi, T., Chabbert, Y.A., (1973) J. Antibiot. 26:612-614.

Williams, J., Northrop, D., (1976) Biochemistry 15:125:131.

Witchitz, J. L., (1972) J. Antibiot. 25:622-624.

Zahm, et al., Mol. Gen. Genet. 194:188-194 (1984).

Zoller, M. M. et al., Methods Enzymol. 100:468 (1983).

## Claims

1. In a method for transforming and regenerating a plant cell, the improvement which comprises using a selectable marker gene encoding a gentamicin-3-N-acetyltransferase enzyme, said gene being adapted to express the enzyme at a sufficient level to render transformed plant tissue tolerant of normally lethal levels of gentamicin.

2. A selectable plant marker gene comprising in sequence:

(a) a promoter which functions in plant cells to cause the production of RNA;

(b) a DNA coding sequence that causes the production of RNA encoding a bacterial gentamicin-3-N-acetyltransferase enzyme; and

(c) a 3′ non-translated region which function in plant cells to cause the addition of polyadenylate nucleotides to the 3′ end of the RNA.

3. A marker gene of Claim 2 in which the promoter is the CaMV35S promoter.

4. A marker gene of Claim 3 in which the 3′ non-translated region is from a nopaline synthase gene of *Agrobacterium tumefaciens*.

5. A marker gene of Claim 4 in which the DNA coding sequence that causes production of a gentamicin-3-N-acetyltransferase enzyme is the sequence of Figure 1.

6. A marker gene of Claim 4 in which the DNA coding sequence that causes production of a gentamicin-3-N-acetyltransferase enzyme is the sequence of Figure 8.

7. A method for selecting a transformed plant cell which comprises:

(a) inserting DNA comprising a plant gene encoding a bacterial gentamicin-3-Nacetyltransferase enzyme into the genome of the plant cell; and

(b) selecting a plant cell containing said gene by culturing said cell in the presence of normally lethal levels of gentamicin.

8. A method of Claim 7 in which the DNA is inserted into the plant cell using an *Agrobacterium* based transformation vector.

9. A method of Claim 8 in which the *Agrobacterium* based transformation vector is a co-integrating vector.

10. A method of Claim 8 in which the *Agrobacterium* based transformation vector is a binary vector.

11. A method of Claim 10 in which the vector is pMON857.

12. A plant transformation vector containing a gene of Claim 2.

13. A plant transformation vector of Claim 12 containing a gene of Claim 5.

14. A plant transformation vector of Claim 12 containing a gene of Claim 6.

15. A plant transformation vector of Claim 12 in which the vector is pMON857.

16. An *Agrobacterium tumefaciens* cell containing a plant transformation vector of Claim 12.

17. An *Agrobacterium tumefaciens* cell of Claim 16 containing a vector of Claim 13.

18. An *Agrobacterium tumefaciens* cell of Claim 16 containing a vector of Claim 14.

19. An *Agrobacterium tumefaciens* cell of Claim 16 containing plasmid pMON857.

20. A transformed plant cell containing a gene comprising:

    (a) a promoter which functions in plant cells to cause the production of RNA;

    (b) a DNA coding sequence that causes the production of RNA encoding a bacterial gentamicin-3-N-acetyltransferase enzyme; and

    (c) a 3' non-translated region which functions in plant cells to cause the addition of polyadenylate nucleotides to the 3' end of the RNA.

21. A transformed plant cell of Claim 20 containing a gene of Claim 3.

22. A transformed plant cell of Claim 20 containing a gene of Claim 4.

23. A transformed plant cell of Claim 20 containing a gene of Claim 5.

24. A transformed plant cell of Claim 20 containing a gene of Claim 6.

Coding sequence of the AAC(3)-IV gene

```
    TCGACTGATGTCATCAGCGGTGGAGTGCAATGTCGTGCAATACGAATGGCGAAAAGCCGA
  1 ---------+---------+---------+---------+---------+---------+ 60
    AGCTGACTACAGTAGTCGCCACCTCACGTTACAGCACGTTATGCTTACCGCTTTTCGGCT

                                            MetSerCysAsnThrAsnGlyGluLysProSer -

    GCTCATCGGTCAGCTTCTCAACCTTGGGGTTACCCCCGGCGGTGTGCTGCTGGTCCACAG
 61 ---------+---------+---------+---------+---------+---------+ 120
    CGAGTAGCCAGTCGAAGAGTTGGAACCCCAATGGGGGCCGCCACACGACGACCAGGTGTC

    SerSerValSerPheSerThrLeuGlyLeuProProAlaValCysCysTrpSerThrAla -

    CTCCTTCCGTAGCGTCCGGCCCCTCGAAGATGGGCCCACTTGGACTGATCGAGGCCCTGC
121 ---------+---------+---------+---------+---------+---------+ 180
    GAGGAAGGCATCGCAGGCCGGGGAGCTTCTACCCGGGTGAACCTGACTAGCTCCGGGACG

    ProSerValAlaSerGlyProSerLysMetGlyProLeuGlyLeuIleGluAlaLeuArg -

    GTGCTGCGCTGGGTCCGGGAGGGACGCTCGTCATGCCCTCGTGGTCAGGTCTGGACGACG
181 ---------+---------+---------+---------+---------+---------+ 240
    CACGACGCGACCCAGGCCCTCCCTGCGAGCAGTACGGGAGCACCAGTCCAGACCTGCTGC

    AlaAlaLeuGlyProGlyGlyThrLeuValMetProSerTrpSerGlyLeuAspAspGlu -

    AGCCGTTCGATCCTGCCACGTCGCCCGTTACACCGGACCTTGGAGTTGTCTCTGACACAT
241 ---------+---------+---------+---------+---------+---------+ 300
    TCGGCAAGCTAGGACGGTGCAGCGGGCAATGTGGCCTGGAACCTCAACAGAGACTGTGTA

    ProPheAspProAlaThrSerProValThrProAspLeuGlyValValSerAspThrPhe -

    TCTGGCGCCTGCCAAATGTAAAGCGCAGCGCCCATCCATTTGCCTTTGCGGCAGCGGGGC
301 ---------+---------+---------+---------+---------+---------+ 360
    AGACCGCGGACGGTTTACATTTCGCGTCGCGGGTAGGTAAACGGAAACGCCGTCGCCCCG

    TrpArgLeuProAsnValLysArgSerAlaHisProPheAlaPheAlaAlaAlaGlyPro -

    CACAGGCAGAGCAGATCATCTCTGATCCATTGCCCCTGCCACCTCACTCGCCTGCAAGCC
361 ---------+---------+---------+---------+---------+---------+ 420
    GTGTCCGTCTCGTCTAGTAGAGACTAGGTAACGGGGACGGTGGAGTGAGCGGACGTTCGG

    GlnAlaGluGlnIleIleSerAspProLeuProLeuProProHisSerProAlaSerPro -

    CGGTCGCCCGTGTCCATGAACTCGATGGGCAGGTACTTCTCCTCGGCGTGGGACACGATG
421 ---------+---------+---------+---------+---------+---------+ 480
    GCCAGCGGGCACAGGTACTTGAGCTACCCGTCCATGAAGAGGAGCCGCACCCTGTGCTAC

    ValAlaArgValHisGluLeuAspGlyGlnValLeuLeuLeuGlyValGlyHisAspAla -

    CCAACACGACGCTGCATCTTGCCGAGTTGATGGCAAAGGTTCCCTATGGGGTGCCGAGAC
481 ---------+---------+---------+---------+---------+---------+ 540
    GGTTGTGCTGCGACGTAGAACGGCTCAACTACCGTTTCCAAGGGATACCCCACGGCTCTG

    AsnThrThrLeuHisLeuAlaGluLeuMetAlaLysValProTyrGlyValProArgHis -
```

**FIGURE 1(a)**

```
      ACTGCACCATTCTTCAGGATGGCAAGTTGGTACGCGTCGATTATCTCGAGAATGACCACT
541   ---------+---------+---------+---------+---------+---------+  600
      TGACGTGGTAAGAAGTCCTACCGTTCAACCATGCGCAGCTAATAGAGCTCTTACTGGTGA

        CysThrIleLeuGlnAspGlyLysLeuValArgValAspTyrLeuGluAsnAspHisCys -

      GCTGTGAGCGCTTTGCCTTGGCGGGACAGGTGGCTCAAGGAGAAGAGCCTTCAGAAGGAA
601   ---------+---------+---------+---------+---------+---------+  660
      CGACACTCGCGAAACGGAACCGCCCTGTCCACCGAGTTCCTCTTCTCGGAAGTCTTCCTT

         CysGluArgPheAlaLeuAlaGlyGlnValAlaGlnGlyGluGluProSerGluGlyArg -

      GGTCCAGTCGGTCATGCCTTTGCTCGGTTGATCCGCTCCCGCGACATTGTGGCGACAGCC
661   ---------+---------+---------+---------+---------+---------+  720
      CCAGGTCAGCCAGTACGGAAACGAGCCAACTAGGCGAGGGCGCTGTAACACCGCTGTCGG

        SerSerArgSerCysLeuCysSerValAspProLeuProArgHisCysGlyAspSerPro -

      CTGGGTCAACTGGGCCGAGATCCGTTGATCTTCCTGCATCCGCCAGAGGGCGGGATGCGA
721   ---------+---------+---------+---------+---------+---------+  780
      GACCCAGTTGACCCGGCTCTAGGCAACTAGAAGGACGTAGGCGGTCTCCCGCCCTACGCT

        GlySerThrGlyProArgSerValAspLeuProAlaSerAlaArgGlyArgAspAlaLys -

      AGAATGCGATGCCGCTCGCCAGTCGATTGGCTGAGCTCATGAGCGGAGAACGAGATGACG
781   ---------+---------+---------+---------+---------+---------+  840
      TCTTACGCTACGGCGAGCGGTCAGCTAACCGACTCGAGTACTCGCCTCTTGCTCTACTGC

        AsnAlaMetProLeuAlaSerArgLeuAlaGluLeuMetSerGlyGluArgAspAspVal -

      TTGGAGGGGCAAGGTCGCGCTGATTGCTGGGGCAACACGTGGAGCGGATCGGGGATTGTC
841   ---------+---------+---------+---------+---------+---------+  900
      AACCTCCCCGTTCCAGCGCGACTAACGACCCCGTTGTGCACCTCGCCTAGCCCCTAACAG

        GlyGlyAlaArgSerArgEnd

      TTTCTTCAGCTCGCTGATGATATGCTGACGCCAATGCCGTTTGGCCTCCGACTAACGAAA
901   ---------+---------+---------+---------+---------+---------+  960
      AAAGAAGTCGAGCGACTACTATACGACTGCGGTTACGGCAAACCGGAGGCTGATTGCTTT

      ATCCCGCATTTGGACGGCTGATCCGATTGGCACGGCGGACGGCGAATGGCGGAGCAGACG
961   ---------+---------+---------+---------+---------+---------+  1020
      TAGGGCGTAAACCTGCCGACTAGGCTAACCGTGCCGCCTGCCGCTTACCGCCTCGTCTGC

      CTCGTCCGGGGGCAATGAGATATGAAAAAGCCTGAACTCACCGCGACGTCTGTCGAGAAG
1021  ---------+---------+---------+---------+---------+---------+  1080
      GAGCAGGCCCCCGTTACTCTATACTTTTTCGGACTTGAGTGGCGCTGCAGACAGCTCTTC

      TTTCTGATCGAAAAGTTCGACAGCGTCTCCGACCTGATGCAGCTCTCGGAGGGCGAAGAA
1081  ---------+---------+---------+---------+---------+---------+  1140
      AAAGACTAGCTTTTCAAGCTGTCGCAGAGGCTGGACTACGTCGAGAGCCTCCCGCTTCTT
```

**FIGURE 1(b)**

```
      TCTCGTGCTTTCAGCTTCGATGTAGGAGGGCGTGGATATGTCCTGCGGGTAAATAGCTGC
1141  ---------+---------+---------+---------+---------+---------+ 1200
      AGAGCACGAAAGTCGAAGCTACATCCTCCCGCACCTATACAGGACGCCCATTTATCGACG


      GCCGATGGTTTCTACAAAGATCGTTATGTTTATCGGCACTTTGCATCGGCCGCGCTCCCG
1201  ---------+---------+---------+---------+---------+---------+ 1260
      CGGCTACCAAAGATGTTTCTAGCAATACAAATAGCCGTGAAACGTAGCCGGCGCGAGGGC


      ATTCCGGAAGTGCTTGACATTGGGGAATTCAGCGAGAGCCTGACCTATTGCATCTCCCGC
1261  ---------+---------+---------+---------+---------+---------+ 1320
      TAAGGCCTTCACGAACTGTAACCCCTTAAGTCGCTCTCGGACTGGATAACGTAGAGGGCG


      CGTGCACAGGGTGTCACGTTGCAAGACCTGCCTGAAACCGAACTGCCCGCTGTTCTGCAG
1321  ---------+---------+---------+---------+---------+---------+ 1380
      GCACGTGTCCCACAGTGCAACGTTCTGGACGGACTTTGGCTTGACGGGCGACAAGACGTC
```

**FIGURE 1(c)**

**FIGURE 2**

SYNTHETIC MULTI-LINKER
EcoRI
BamHI
HindIII
BglII

NOS-NPTII'-NOS
BamHI
StuI
Tn7 Spc/Str R
BstEII
HpaI

P 3
TI HOMOLOGY LIH
SmaI
PBR322 ORI

0
(10098)

pMON316

8000
2000
6000
4000

NOPALINE SYNTHASE pTIT37
RIGHT BORDER
BamHI

FIGURE 3

0289478

**FIGURE 4**

## CaMV 35S PROMOTER

Filled EcoRI
1 |
GAATTAATTCCCGATCCTATCTGTCACTTCATCAAAAGGACAGTAGAAAAGGAAGGTGGC 60

ACTACAAATGCCATCATTGCGATAAAGGAAAGGCTATCGTTCAAGATGCCTCTGCCGACA 120

GTGGTCCCAAAGATGGACCCCCACCCACGAGGAGCATCGTGGAAAAAGAAGACGTTCCAA 180

CCACGTCTTCAAAGCAAGTGGATTGATGTGATATCTCCACTGACGTAAGGGATGACGCAC 240

TATA
AATCCCACTATCCTTCGCAAGACCCTTCCTCTATATAAGGAAGTTCATTTCATTTGGAGA 300

5' mRNA
|                          332
GGACACGCTGAAATCACCAGTCTCTCTCTACA

### SYNTHETIC MULTI-LINKER

BglII  ClaI SmaI KpnI SalI EcoRI
|      |    |    |  | |    |
AGATCTATCGATTCCCGGGTACCTCGAGAATTCCC

## NOS 3'

368                                                    420
GATCGTTCAAACATTTGGCAATAAAGTTTCTTAAGATTGAATCCTGTTGCCGG

480
TCTTGCGATGATTATCATATAATTTCTGTTGAATTACGTTAAGCATGTAATAATTAACAT

3' End mRNA
| |  ||||||| 540
GTAATGCATGACGTTATTTATGAGATGGGTTTTTATGATTAGAGTCCCGCAATTATACAT

600
TTAATACGCGATAGAAAACAAAATATAGCGCGCAAACTAGGATAAATTATCGCGCGCGGT

HindIII
641| 648
TCATCTATGTTACTAGATCggggatccgtcgacctgcagccaagctt

**FIGURE 5**

**FIGURE 6**

**FIGURE 7**

0289478

Coding sequence of the AAC(3)-III gene

```
    TCGCGATGCATACGCGGAAGGCAATAACGGAGGCAATTCGAAAACTCGGAGTCCAAACCG
  1 ---------+---------+---------+---------+---------+---------+ 60
    AGCGCTACGTATGCGCCTTCCGTTATTGCCTCCGTTAAGCTTTTGAGCCTCAGGTTTGGC

      MetHisThrArgLysAlaIleThrGluAlaIleArgLysLeuGlyValGlnThrGly -

    GTGACCTGTTGATGGTGCATGCCTCACTTAAAGCGATTGGTCCGGTCGAAGGAGGAGCGG
 61 ---------+---------+---------+---------+---------+---------+ 120
    CACTGGACAACTACCACGTACGGAGTGAATTTCGCTAACCAGGCCAGCTTCCTCCTCGCC

      AspLeuLeuMetValHisAlaSerLeuLysAlaIleGlyProValGluGlyGlyAlaGlu -

    AGACGGTCGTTGCCGCGTTACGCTCCGCGGTTGGGCCGACTGGCACTGTGATGGGATACG
121 ---------+---------+---------+---------+---------+---------+ 180
    TCTGCCAGCAACGGCGCAATGCGAGGCGCCAACCCGGCTGACCGTGACACTACCCTATGC

      ThrValValAlaAlaLeuArgSerAlaValGlyProThrGlyThrValMetGlyTyrAla -

    CGTCGTGGGACCGATCACCCTACGAGGAGACTCTGAATGGCGCTCGGTTGGATGACAAAG
181 ---------+---------+---------+---------+---------+---------+ 240
    GCAGCACCCTGGCTAGTGGGATGCTCCTCTGAGACTTACCGCGAGCCAACCTACTGTTTC

      SerTrpAspArgSerProTyrGluGluThrLeuAsnGlyAlaArgLeuAspAspLysAla -

    CCCGCCGTACCTGGCCGCCGTTCGATCCCGCAACGGCCGGGACTTACCGTGGGTTCGGCC
241 ---------+---------+---------+---------+---------+---------+ 300
    GGGCGGCATGGACCGGCGGCAAGCTAGGGCGTTGCCGGCCCTGAATGGCACCCAAGCCGG

      ArgArgThrTrpProProPheAspProAlaThrAlaGlyThrTyrArgGlyPheGlyLeu -

    TGCTGAATCAATTTCTGGTTCAAGCCCCCGGCGCGCGGCGCAGCGCGCACCCCGATGCAT
301 ---------+---------+---------+---------+---------+---------+ 360
    ACGACTTAGTTAAAGACCAAGTTCGGGGGCCGCGCGCCGCGTCGCGCGTGGGGCTACGTA

      LeuAsnGlnPheLeuValGlnAlaProGlyAlaArgArgSerAlaHisProAspAlaSer -

    CGATGGTCGCGGTTGGTCCGCTAGCTGAAACGCTGACGGAGCCTCACGAACTCGGTCACG
361 ---------+---------+---------+---------+---------+---------+ 420
    GCTACCAGCGCCAACCAGGCGATCGACTTTGCGACTGCCTCGGAGTGCTTGAGCCAGTGC

      MetValAlaValGlyProLeuAlaGluThrLeuThrGluProHisGluLeuGlyHisAla -

    CCTTGGGGGAAGGGTCGCCCGTCGAGCGGTTCGTCCGCCTTGGCGGGAAGGCCCTGCTGT
421 ---------+---------+---------+---------+---------+---------+ 480
    GGAACCCCCTTCCCAGCGGGCAGCTCGCCAAGCAGGCGGAACCGCCCTTCCGGGACGACA

      LeuGlyGluGlySerProValGluArgPheValArgLeuGlyGlyLysAlaLeuLeuLeu -

    TGGGTGCGCCGCTAAACTCCGTTACCGCATTGCACTACGCCGAGGCGGTTGCGGATATCC
481 ---------+---------+---------+---------+---------+---------+ 540
    ACCCACGCGGCGATTTGAGGCAATGGCGTAACGTGATGCGGCTCCGCCAACGCCTATAGG

      GlyAlaProLeuAsnSerValThrAlaLeuHisTyrAlaGluAlaValAlaAspIlePro -
```

**FIGURE 8(a)**

```
     CCAACAAACGATGGGTGACGTATGAGATGCCGATGCTTGGAAGAAACGGTGAAGTCGCCT
541  ---------+---------+---------+---------+---------+---------+ 600
     GGTTGTTTGCTACCCACTGCATACTCTACGGCTACGAACCTTCTTTGCCACTTCAGCGGA

     AsnLysArgTrpValThrTyrGluMetProMetLeuGlyArgAsnGlyGluValAlaTrp -

     GGAAAACGGCATCAGAATACGATTCAAACGGCATTCTCGATTGCTTTGCTATCGAAGGAA
601  ---------+---------+---------+---------+---------+---------+ 660
     CCTTTTGCCGTAGTCTTATGCTAAGTTTGCCGTAAGAGCTAACGAAACGATAGCTTCCTT

     LysThrAlaSerGluTyrAspSerAsnGlyIleLeuAspCysPheAlaIleGluGlyLys -

     AGCCGGATGCGGTCGAAACTATAGCAAATGCTTACGTGAAGCTCGGTCGCCATCGAGAAG
661  ---------+---------+---------+---------+---------+---------+ 720
     TCGGCCTACGCCAGCTTTGATATCGTTTACGAATGCACTTCGAGCCAGCGGTAGCTCTTC

     ProAspAlaValGluThrIleAlaAsnAlaTyrValLysLeuGlyArgHisArgGluGly -

     GTGTCGTGGGCTTTGCTCAGTGCTACCTGTTCGACGCGCAGGACATCGTGACGTTCGGCG
721  ---------+---------+---------+---------+---------+---------+ 780
     CACAGCACCCGAAACGAGTCACGATGGACAAGCTGCGCGTCCTGTAGCACTGCAAGCCGC

     ValValGlyPheAlaGlnCysTyrLeuPheAspAlaGlnAspIleValThrPheGlyVal -

     TCACCTATCTTGAGAAGCACTTCGGAGCCACTCCGATCGTGCCAGCACACGAAGCCGCCC
781  ---------+---------+---------+---------+---------+---------+ 840
     AGTGGATAGAACTCTTCGTGAAGCCTCGGTGAGGCTAGCACGGTCGTGTGCTTCGGCGGG

     ThrTyrLeuGluLysHisPheGlyAlaThrProIleValProAlaHisGluAlaAlaGln -

     AGCGCTCTTGCGAGCCTTCCGGTTAGAGGCCGTCGAC
841  ---------+---------+---------+------- 877
     TCGCGAGAACGCTCGGAAGGCCAATCTCCGGCAGCTG

     ArgSerCysGluProSerGlyEnd
```

**FIGURE 8(b)**

FIGURE 9

FIGURE 10